# EUROPEAN PATENT APPLICATION

(11) **EP 1 498 117 A1**
(43) Date of publication of application: **19.01.2005**
(21) Application number: 03016056.8
(22) Date of filing: 15.07.2003
(51) Int. Cl.: A61K 9/48

(54) **A pharmaceutical gelatin-capsule or gelatin containing capsule preparation showing an improved stability, a process for making the capsules and a process for improving the stability of gelatin-capsules**

(71) Applicant: Pfizer GmbH Arzneimittelwerk Gödecke, 79090 Freiburg (DE)
(72) Inventor: Knapp, Armin, Dr., 79288 Gottenheim (DE); Schreder, Sven, Dr., 79104 Freiburg (DE)
(74) Representative: Tesch, Rudolf

(57) **Abstract**

The invention concerns a pharmaceutical gelatin capsule composition consisting of a) an active composition comprising pharmaceutical adjuvants and/or at least one active ingredient, b) a barrier-composition comprising one or more quenching agents, the quenching agent(s) being able to chemically bind volatile impurities initiating the cross-linking of gelatin, by means of a substantially irreversible chemical reaction after said impurities have been released by said adjuvants and/or active compound(s) and c) a gelatin-capsule shell or a gelatine containing capsule shell surrounding the active composition and the barrier-composition. The barrier-composition is positioned inside the capsule shell in a way forcing said volatile impurities to contact and penetrate the surface of the barrier-composition before said impurities are able to contact the inner surface of the gelatin-capsule shell of the pharmaceutical composition. The invention is also directed to a process for preventing and/or reducing the cross-linking of gelatin in gelatin-capsule shells and to a method of improving the stability of pharmaceutical gelatine-capsule preparations.
It has been found that gelatine and/or amino-acids may be used as quenching agents binding volatile impurities improving the stability of pharmaceutical gelatine capsules.

## Description

The application concerns a pharmaceutical gelatin-capsule or gelatin containing capsule preparation showing an improved stability especially under "stressing" storage conditions. The application further concerns a process for preparing the improved gelatin-capsule or gelatin containing capsule preparation and a process for improving the storage stability of gelatin-capsule preparations.

The known status of technology in the manufacture of capsules with controlled release of active compounds comprises the coating of particulate compositions comprising active compounds such as pellets, granulates, mini-tablets, mini- or microcapsules or caplets with commercially-available aqueous dispersions such as ammonio methacrylate copolymers, Type A (Eudragit® RL 30 D) and Type B (Eudragit® RS 30 D) or also with other aqueous dispersions of retarding polymers under concurrent or subsequent drying and subsequent filling into commercially-available empty capsules, in most cases gelatin-capsules. For reasons of environmental and work safety and of manufacturing costs the use of organic solvents should be avoided. Aqueous processes are therefore preferred.

Aqueous dispersions usually are stabilized with preservatives, however, mixtures of different dispersions such as the ammonio methacrylate copolymer-types A and B can be processed without preservatives by using them as ready-to-use organic solutions (Eudragit© RL 12,5; Eudragit® RS 12,5) in isopropanol and acetone, or as solid compositions (Eudragit® RL 100, Eudragit® RS 100) which must be dissolved in organic solvents immediately prior to use. This organic coating process is coupled with considerable cost in equipment needed to protect the operators and the environment from contamination and solvent explosions. A lower-cost alternative is the direct pressing of mini-tablets or caplets containing the active compounds together with ammonio methacrylate copolymers (Eudragit® RL/PO, Eudragit® RS PO) as solid mixtures by direct pressing followed by the usual encapsulation. However, the direct pressing method sets requirements of form, size and tablettability and is, therefore, not as versatile as the coating process with aqueous dispersions described above. The above described mixtures are used to set up or adjust a retarding profile in order to obtain the desired activity profile for a pharmaceutical capsule.

In order to attain a fast-acting initial dose, the retarded particulate compositions coated e.g. with ammonio methacrylate copolymers may be additionally coated with a non-retarding layer of one or more active substances, or the particulate compositions coated may be combined with immediate-release compositions containing the actives or they may combined with immediate-release powders containing the active compound(s) in the same capsule. Moreover, particulate compositions with a different active release profile may be combined in one capsule. All of these very variable compositions are finally filled into empty capsules, mostly gelatin-capsules in the desired dosage and stored for a longer time before use.

It has been reported [Pharmaceutical Technology, Murthy et al., Part I, 13(3) pp. 72-86 (1989)] that pharmaceutical preparations based on gelatin capsules show undesired effects of interaction between storage conditions and formulation factors. There was for example a case reported that involved a lack of clinical efficacy of phenytoin capsules because of improper storage. These capsules were stored in a humid environment and displayed in-vitro disintegration and dissolution characteristics markedly slower than those of control samples of a freshly prepared capsule.

In [Pharmaceutical Technology, Murthy et al., Part II, 13(6) pp. 53-58 (1989)] it has further been published that not only humidity but also the influence of light may cause adverse effects on the dissolution rates of hard gelatin capsules. In further investigations the authors of the above publications, Murthy et al., considered chemical influences of ingredients such as minor contents of aldehydes, sulfonic acids, hydrogen peroxide and/or special dyes like FD&C Rot Nr.3 and Nr. 40 and some other certified colors present in the active compounds and/or adjuvants as possible triggers of a so called "cross-linking" process of gelatin. These additives under adverse conditions severely impaired the dissolution of capsule shells after storage.

In the Journal of Pharmaceutical Sciences,83, no.7, (1994) gelatin is described as a mixture of water-soluble proteins derived primarily from collagen. Gelatin undergoes conformational change and cross-linking when stored under high-humidity conditions and/or high temperatures as was demonstrated by active compounds stored in the gelatin capsules such as gemfibrozil, hydrochlorothiazide and diphenhydramine-hydrochloride. The cross-linking process caused formation of a swollen, rubbery, water-insoluble gelatin membrane (pellicle) during dissolution testing. This water-insoluble gelatin film acts as a barrier, restricting the drug release. The publication has discussed a theory, how the influence of humidity in the cross-linking process could work and the authors believed that indirectly catalyzed imide-formation is starting the subsequent cross-linking reaction. By this reason this kind of reaction has already been used to prepare gelatin-capsules which are resistant to gastric juice.

A conclusion of these considerations is that formaldehyde is the main cross-linking agent responsible for the loss of solubility of gelatin capsules, based on the assumption that this agent reacts with the epsilon-amino-function of lysine and the guanidine-function of arginine residues both present in gelatin.

In later studies [Journal of Pharmaceutical Sciences Vol 83, No.7 1994] softeners, preservatives, lipids, polyethoxylated compounds like polyethylene glycol, ethers of PEG and aliphatic alcohols and phenols, polyethoxylated glycerides and non-ionic surface active compounds (polysorbate ester, unsaturated fatty acids), which all form aldehydes in their degradation due to autoxidation, have been discussed as further possible "triggers".

The inventors' own investigations proved that gelatin capsules filled with particulate compositions coated with aqueous dispersions of ammonio methacrylate copolymer-type A (Eudragit® RL 30 D) or with mixtures of Types A and B (Eudragit® RS 30 D) or with compositions produced from solid ammonio methacrylate copolymer Type A (Eudragit® RL PO) are cross-linked during storage, which delays the dissolution rate of the capsule shell and thus exerts uncontrolled changes on the programmed release of the capsule content. The change in programmed release may occur as a delay in the initial release of active substances, as a further delay in retarded release of active substance(s), or by interaction of formulation ingredients (e.g. by interaction of soluble, rapid-release anionic components with the ammonio methacrylate copolymers) as accelerated release of retarded active ingredients.

According to the above discussions the cross-linking process, especially after a longer time of storage of filled gelatin capsules under humid and/or higher temperature conditions results in a markedly slower disintegration and a retarded release of the active compounds from such capsule preparations.

While the influence of gelatin cross-linking could be verified by convincing in-vitro studies, in-vivo investigations suggested that cross-linking is less harmful as expected for the in-vivo performance and bioequivalence of retarded pharmaceutical preparations [Pharmaceutical Research, Vol. 15, No.7, p.1030 (1998)].

On the other hand, it must be considered, especially if the capsule contains immediate to release components, that the cross-linking effect is responsible for release delays of up to 12 minutes, while the standard disintegration time of a freshly prepared capsule is only about 3 minutes. Strongly cross-linked gelatin capsules may be even totally resistant to gastric juice. In less cross-linked preparations the release time of the pharmacologically active particulate components is still delayed by 5 to 10 minutes.

In the case of preferably fast acting pharmaceuticals such as analgesics or migraine preparations, fast action is required and a considerably delayed disintegration time is certainly not acceptable.

Furthermore it was recently found that in gelatin capsules or gelatin-containing capsules filled with compositions in the form of particles of different content of additives, unwanted interactions between the content of these compositions may take place because, in the case of a strong cross-linking effect on parts of the gelatin shell, the capsule shell may partially leak relatively early e.g. as early as 3 minutes after being in contact with gastric juice, while the capsule, due to the cross-linked parts of the shell, maintains its main structure and does not allow the release of particulate components for another 10 minutes before the complete breakup of the capsule-shell.

In the "time-gap" of about 7 minutes, fast releasing components dissolve almost immediately inside the capsule-shell and form highly concentrated solutions of soluble ingredients which have sufficient time to interact severely with still intact retarded particulate components. Osmotic influences and different permeabilities of e.g. chloride and sulphate anions may cause severe damage to a semipermeable layer of the retarded components, and these undesired interactions change the release characteristics and bioavailability of the capsule in an unacceptable degree.

An example for such interactions may be Eletriptan [US-A 2002/0034545], a highly active drug for the prevention of migraine recurrence, which is based on a short acting and a retarded particulate component of the active compound. Only recently it was found that the chloride and sulphate anions of different ammonio-methacrylates, in the dry capsule separated from each other in different particulate components, after stressing, especially under humid and high temperature storage conditions, within a very short time after being in contact with gastric juice, interacted with the capsules and thus showed an unacceptably accelerated release of the retarded components and strongly varying disintegration times of the capsule shell. Such interactions may therefore/be responsible for high losses due to a considerable amount of discarded capsules showing release properties outside the allowed range.

These findings demonstrate that cross-linking is far more critical for the release profile of active compounds in-vivo and for the bioavailability of drugs based on gelatin-capsules than it was hitherto expected and predicted [George Digenis (Journ. of Pharm.Sci. Vol.83, No.7 {1994})] and [Pharm. Research Vol. 17, No. 8, p 962 ff. (2000)].

As a consequence of the above considerations, the undesired cross-linking of gelatin-capsule or gelatin containing capsule shells should be avoided whenever possible.

The purpose of the present invention is to provide means allowing the control and prevention of the cross-linking-process in hard- and soft-gelatin capsules or gelatin-containing capsules filled with pharmaceutical preparations containing and releasing compounds which trigger or promote the cross-linking process.

The task of the invention has been resolved by means that quench or chemically absorb such volatile triggering impurities before they are able to reach the inner surface of the gelatin-capsule shell and thus prevent any a further cross-linking activity of the volatile impurities with the capsule-forming gelatin.

The present invention enables storage of the capsule product in customary packaging over a long time period, with no significant change in the dissolution of the capsule shell and/or the release of the active ingredient.

In order to further improve the stability by preventing the cross-linking of the gelatin-shell, volatile impurities of the capsule-filling triggering the cross-linking of the gelatin shell as for example aldehyde impurities should be kept at a level where the unwanted reaction in the gelatin-shell is not triggered. The most important trigger substances are volatile aldehydes, especially formaldehyde or lower aliphatic aldehydes with up to 6 carbon atoms. The aldehyde content of the filling should be less than 10 ppm, preferably less than 5 ppm and most preferably less than 2 ppm referred to the gelatin-shell.

The removal of volatile impurities effecting or triggering the cross-linking reaction in the gelatin shell as for example aldehyde impurities may be achieved by vacuum treatment (less than 100 mbar, preferably 1-20 mbar) of the retard pellets at raised temperatures (25 - 50°C, preferably 35 - 45°C) or by keeping the retard pellets in a clean air or nitrogen flow at raised temperatures (30-50°C, preferably 35-45°C) in a fluidized bed unit, preferably with controlled inlet air humidity. In the case of volatile aldehyde impurities such impurities should not be higher than 5 ppm, preferably less than 2 ppm in relation to the gelatin-capsule or gelatin containing capsule shell.

As a consequence of the above the following means support the preparation of pharmaceutical gelatin-capsule preparations according to the present invention:
a.) Follow-up treatment of the filling preparations or adjuvants including or not including the active compounds in order to reduce the content of volatile trigger-substances especially aldehyde impurities to less than 5 ppm, preferably less than 2 ppm in relation to the gelatin capsule shell. The removal of volatile aldehyde impurities from e.g. ammonio methacrylate copolymer Type A and/or retard pellets may be achieved by vacuum treatment (less than 100 mbar, preferably 1 - 20 mbar) of the retard pellets at raised temperatures (25 - 50°C, preferably 35 - 45°C) or by keeping the retard pellets in a clean and dry air or nitrogen flow at raised temperatures (30-50°C, preferably 35-45°C) in a fluidized bed unit, preferably with controlled inlet air humidity providing an air of less than 20% relative humidity.
b.) The application of a lipophilic protective layer consisting of or containing lipids (e.g. glycerinmonostearate) or waxing on the retarding layer with ammonio methacrylate copolymer Type A.
c.) The application of a protective layer consisting of or containing amines, amino-acids, oligo- or polypeptides on the retarding layer with ammonio methacrylate copolymer Type A.

A first object of the present invention is a pharmaceutical composition in gelatin-capsule form consisting of:
a) An active composition comprising pharmaceutical adjuvants and/or at least one active ingredient,
b) a barrier-composition comprising one or more quenching agents, the quenching agent being able to chemically bind volatile impurities initiating the cross-linking of gelatin by a substantially irreversible chemical reaction after said impurities have been released by said adjuvants and/or active compound(s),
c) a gelatin-capsule shell surrounding the active composition and the barrier-composition
wherein the inner barrier-composition is positioned inside the capsule shell in a way forcing, the volatile impurities to contact, preferably penetrate, the barrier before said
impurities are able to contact the inner surface of the gelatin-capsule shell of the pharmaceutical composition.

A second object of the present invention is a process for improving the stability of a pharmaceutical gelatin-capsule preparation by preventing and/or reducing the cross-linking of gelatin in the gelatin capsule shell initiated by volatile impurities released by components of the capsule filling, comprising the steps:
a) preparing a barrier-composition comprising at least one quenching-agent which is able to chemically bind volatile impurities initiating the cross-linking of gelatin, by a substantially irreversible chemical reaction,
b) positioning said barrier-composition between the compositions or compounds releasing said volatile impurities and the inner surface of the gelatin-capsule shell in a way that said volatile impurities are forced to contact and penetrate the surface of said barrier composition before said volatile impurities are able to contact the inner surface of the gelatin-capsule shell.

The quenching-reagent in its broadest sense may be a compound that anticipates the unwanted cross-linking reaction occurring in the gelatin-capsule shell. Depending on the nature of the volatile impurity such quenching-reagent may be gelatin and/or an amino-acid such as glycin or asparagin.

The quenching-agent may be applied in several ways to form a barrier between the source of the volatile impurities and the outer gelatin-capsule or gelatin containing capsule shell.

Preferred are capsule preparations, wherein the barrier-composition is positioned on the inner surface of the gelatine or gelatin-containing shell.

Also preferred is a capsule preparation, wherein the barrier-composition is positioned on the outer surface of the active composition.

Gelatin containing capsule-shells comprise pharmaceutically acceptable shell materials consisting of readily water soluble polymers that may also contain adjuvants. The gelatine content must be sufficiently high to be receptive to the described cross-linking reactions. Depending on the conditions and adjuvants this may be in the range of a gelatin content of almost 100 down to 20 weight%.

One method is the addition of amino acids or gelatin to one of the methacrylate dispersions or powders described above and - together with the active ingredients - the formation a coated or mixed and pressed composition. The immediate contact of the quenching agent with the ingredients of the composition forms a barrier-layer all around thel particles of the composition including the methacrylate particles. This makes sure that any volatile impurity released from the compositions in the capsule will be bound to the quenching-additive before the volatile impurity is able to react with the gelatin of the outer shell.

A further method is using a water soluble outer layer containing said quenching agent around substantially all of the pharmaceutical particles described above, such as pelets, mini-tablets, caplets or granules. The, particulate barrier-composition may be prepared in the form of a water-soluble outer coating layer around pharmaceutical core particles containing the active compound(s) and semipermeable film forming materials such as, for example, acrylic copolymers with methacrylates. The core particles especially under "stressing" storing conditions may release volatile impurities in the sense of the present invention. The outer layer may comprise a water soluble film forming material such as the hydrophilic polymers hydroxypropyl-methylcellulose, hydroxypropyl-cellulose, polyvinyl-alcohol, or any combination thereof and as the barrier-forming substances gelatin or an amino-acid in an amount of 1 % to 60% by weight of said coating composition.

The highest possible amount of gelatin should be applied with the proviso that the stability and solubility of the final coating is not unacceptably deteriorated. This final coating may account from 1 to 25%, preferably 1 to 15% by weight of the final core product.

Immediate and modified-release pellets were prepared according to WO-A 02/09675 with protective coating levels from 1 to 10, preferably 1 to 3 weight-%. When the beads obtained were filled into hard gelatin capsule shells using automatic capsule-filling machines with pellet-filling stations, severe scratching of the machine parts was heard. The process had to be stopped and machine parts of the capsule-filling stations had to be replaced because of deep scratching traces on the surface.

Surprisingly it was found, that protective coating levels between 10 and 25 weight-%, preferably 10 to 15 weight-% provided for capsule-filling properties allowing the filling of commercial batch sizes without any technical difficulties and avoiding the damages described above.

The barrier-composition may also be added in a way based on conventional methods as for example described in US-A 2002/0034545, paragraph [005] to [007], for compositions, wherein an inner core is coated first with a water-insoluble,permeable or semipermeable coating including one or more acrylic copolymer(s) containing trimethyl-ammonium-metacrylate groups, whereafter the coated core is provided with a second water soluble coating layer comprising the barrier-agent as described above, e.g. gelatin or an amino-acid. This combination of layers can be varied in a way that the barrier forming agent is added to the first semipermeable layer or even to both layers. The term "water insoluble , semipermeable or permeable coating" used in the definition of the particulate compositions above means a coating which is resistant to degradation under aqueous conditions encountered in the gastrointestinal tract for at least 24 hours but which is semipermeable or permeable for the active substances, as e.g. Eletriptan or corresponding salts, when in contact with an aqueous medium, so as to allow the passage of dissolved drugs through the permeable or semipermeable coating. The rate of drug release will vary with the coating thickness which is typically from 10 to 100 microns, preferably from 40 to 80 microns.
In another preparation the barrier-composition is prepared by admixing the barrier-component as a solid powder or granulate or as a film forming suspension to the active component and possible further adjuvants in an amount, that the above volatile impurities have to pass the quenching material and by doing so are completely bound to the barrier-compound(s) and thus prevented to be released from the particles and to get to the inner surface of the gelatin-capsule or gelatin containing capsule.

A further application of the invention is the use of the above quenching agent in "cap-lets" according to EP-A 0 891 180. Such caplets may be included into gelatin-capsule shells. They comprise a coating composition comprising cellacephate, polyvinyl acetate-phthalate, methacrylic acid polymers, hypromellose, phthalate, hydroxyalkyl methylcellulose phthalates or mixtures thereof. The barrier may be prepared by an additional water-soluble layer containing an amino acid. In the case of caplets it is also possible to add e.g. gelatin to the above coating before placing the caplet in the capsule shell. The film forming agent in the case of caplets is used in an amount of about 20% of weight. Glidants and or adhesives for fixing the caplet in the shell may be added. If gelatin and/or an amino-acid is added to the film forming material as barrier-substances, the amount may be 1 to 50 weight % of the film forming material.
It is obvious to the person skilled in the pharmaceutical art, that the ingredients of the total capsule contents as well as the capsule forming gelatin must be as clean as possible before the capsule and/or its contents are finally prepared and composed.

The present invention thus provides a storage stable gelatin-capsule or gelatin containing capsule preparation which in a costumary packaging may be stored over a long time even under the describes stress conditions it is under relatively high temperatures above 30° C and a high humidity of more than 50%.

The capsules of the invention maintain the bioavailability profile that is substantially identical to the initial product before storage. The advantages of the new preparation may be supported by an additional protective lipophilic layer applied to the particulate capsule components. Such layer may be added in the usual way in a separate coating step. HPMC is easily soluble in water and body liquids. This guarantees minimal influences of the desired profile of drug release and in its dry form, during storage, the complete quenching of volatile impurities before they are able to contact the inner surface of the gelatin capsule or gelatine containing capsule shell.

The acrylic copolymer(s) containing trimethylammoniumethylmethacrylate groups included in the water-insoluble, permeable coating is/are preferably selected from the Eudragit RL® and Eudragit RS® copolymers mentioned above. These copolymers contain chloride counter-ions.

The water-insoluble, permeable or semipermeable coating may include one or more additional substances other than an acrylic copolymer containing trimethylammo-nium-ethyl-methacrylate groups, such as a plasticiser (e.g. an acetylated monoglyc-eride, triethyl citrate, acetyltriethyl citrate, tributyl citrate, acetyltributyl citrate, another citrate ester, dibutyl phthalate, diethyl phthalate, another phthalate ester, diethyl se-bacate, dibutyl sebacate, diethyl fumarate, diethyl succinate, a polyethylene glycol, glycerol, sesame oil, a lanolin alcohol or triacetin), an anti-tacking agent (e.g. talc, calcium stearate, colloidal silicon dioxide, glycerin, magnesium stearate, mineral oil, a polyethylene glycol, zinc stearate, aluminium stearate or glycerol monostearate), a wetting agent (e.g. sodium lauryl sulphate, stearyl alcohol, acacia, benzalkonium chloride, cetomacrogol emulsifying wax, cetostearyl alcohol, cetyl alcohol, cholesterol, diethanolamine, sodium stearate, glycerol monostearate, hydroxypropylcellulose, a lanolin alcohol, triethanolamine, lecithin, poloxamer, a polyoxyethylene alkyl ether, a sorbitan ester, a stearyl alcohol or simethicone) or a water insoluble polymer (e.g. ethylcellulose, cellulose acetate or a polymethacrylate copolymer). A preferred plasticiser is trieth-ylcitrate, a preferred anti-tacking agent is talc and a preferred wetting agent is sodium lauryl sulphate.

## Claims

1. A pharmaceutical composition in a gelatin-capsule or gelatin-containig capsule comprising:
a) an active composition comprising pharmaceutical adjuvants and/or at least one active ingredient,
b) a barrier-composition comprising one or more quenching agents, the quenching agent(s) being able to chemically bind volatile impurities initiating the cross-linking of gelatin, by means of a substantially irreversible chemical reaction of said quenching agent(s) with said volatile impurities after said volatile impurities have been released by said adjuvants and/or active compound(s),
c) a gelatin-capsule shell surrounding the active composition and the barrier-composition,
wherein the barrier-composition is positioned inside the capsule shell in a way forcing said volatile impurities to contact and penetrate the surface of the barrier-composition before said impurities are able to contact the inner surface of the gelatin-capsule shell of the pharmaceutical composition.

2. A pharmaceutical composition according to Claim 1 wherein the gelatin-capsule is a soft gelatin-capsule.

3. A pharmaceutical composition according to Claims 1 or 2, wherein the gelatin-capsule is a hard gelatin-capsule.

4. A pharmaceutical composition according to Claims 1 to 3, wherein the quenching agent comprises gelatin and/or an amino-acid.

5. A pharmaceutical composition according to Claim 4, wherein the quenching agent comprises gelatin.

6. A pharmaceutical composition according to Claim 4, wherein the quenching agent comprises a C² to C⁶ amino acid.

7. A pharmaceutical composition according to Claim 6, wherein the amino-acid is glycine or aspartic acid.

8. A pharmaceutical composition according to Claims 1 to 7, wherein the barrier-composition is positioned as a separate layer on the inner surface of the gelatine or gelatin-containing shell.

9. A pharmaceutical composition according to Claims 1 to 7, wherein the barrier-composition is positioned on the outer surface of the active composition.

10. A process for preventing and/or reducing the cross-linking of gelatin or gelatin-containing capsule shells which is initiated by volatile impurities released by the components of the capsule filling, comprising the steps:
a) preparing a barrier-composition comprising at least one quenching-agent which is able to chemically bind volatile impurities initiating the cross-linking of gelatin by a substantially irreversible chemical reaction and
b) positioning said barrier-composition between the source(s) releasing said volatile impurities and the inner surface of the gelatin-capsule shell in a way that said volatile impurities are forced to contact and penetrate the surface of said barrier composition before said volatile impurities are able to contact the inner surface of the gelatin-capsule shell.

11. A process according to Claim 10, wherein the gelatin-capsule is a soft gelatin-capsule.

12. A process according to Claim 10, wherein the gelatin-capsule is a hard gelatin-capsule.

13. A process according to Claims 10 to 12, wherein the quenching agent comprises gelatine and/or a C2 to C⁶ amino acid.

14. A process according to Claim 10 to13, wherein the quenching agent is gelatin.

15. A process according to Claim 10 to 13, wherein the quenching agent is a C² to C⁶ amino-acid.

16. A process according to Claim 10 to 15, wherein the amino-acid is glycine or aspartic acid.

17. A process according to Claims 10 to 16, wherein the barrier-composition is positioned as a separate layer on the inner surface of the gelatine or gelatin-containing shell.

18. A process according to Claims 10 to 16, wherein the barrier-composition is positioned on the outer surface of the active composition.

19. A method of improving the stability of pharmaceutical gelatine- or gelatine- containing capsule preparation **characterized by** the following steps:
a) preparing a barrier-composition comprising at least one quenching-agent which is able to chemically bind volatile impurities released by components of the capsule filling which initiate the cross-linking of gelatin by a substantially irreversible chemical reaction of said quenching agent with said volatile impurity and
b) positioning said barrier-composition between the source(s) releasing said volatile impurities and the inner surface of the gelatin-capsule shell in a way that said volatile impurities are forced to contact and penetrate the surface of said barrier composition before said volatile impurities are able to contact the inner surface of the gelatin-capsule shell.

20. A method according to Claim 19, wherein the barrier-composition is positioned on the inner surface of the gelatine or gelatin-containing shell.

21. A method according to Claim 19, wherein the barrier-composition is positioned on the outer surface of the active composition.

22. Use of gelatine and or amino-acids as quenching agents for improving the stability of pharmaceutical gelatine capsules.
